# EUROPÄISCHE PATENTSCHRIFT

(11) **EP 0 986 548 B1**
(45) Veröffentlichungstag und Bekanntmachung des Hinweises auf die Patenterteilung: **30.07.2003**
(21) Anmeldenummer: 98934857.8
(22) Anmeldetag: 28.05.1998
(51) Int. Cl.: C07D 257/02, C07D 405/04

(54) **HERSTELLUNG VON MONO- UND 1,7-BIS-N-HYDROXYALKYL-CYCLEN UND LITHIUM-SALZ-KOMPLEXEN**
PRODUCTION OF MONO AND 1,7-BIS-N-HYDROXYALKYL-CYCLENE AND LITHIUM SALT COMPLEXES
PREPARATION DE COMPLEXES DE MONO- ET 1,7-BIS N-HYDROXYALKYL-CYCLENE ET DE SEL DE LITHIUM

(30) Priorität: 02.06.1997 DE 19724186
(43) Veröffentlichungstag der Anmeldung: 22.03.2000
(73) Patentinhaber: Schering Aktiengesellschaft, 13353 Berlin (DE)
(72) Erfinder: PETROV, Orlin, D-14199 Berlin (DE); BLASZKIEWICZ, Peter, D-12167 Berlin (DE)
(86) Internationale Anmeldenummer: DE9801523
(87) Internationale Veröffentlichungsnummer: WO98055467

(56) Entgegenhaltungen:
- EP-A- 0 485 045
- EP-A- 0 545 511
- EP-A- 0 596 586
- WO-A-93/24469
- WO-A-94/27977
- CHEMICAL ABSTRACTS, vol. 124, no. 25, 17. Juni 1996 Columbus, Ohio, US; abstract no. 343242, FORMANOVSKY A.A. : "One-stage monosubstitution in cyclen" XP002077999 & SYNTH. COMMUN., Bd. 26, Nr. 8, 1996, Seiten 1595-1603,

## Beschreibung

Die Erfindung betrifft Verfahren zur Mono- und 1,7-Bis-N-β-Hydroxyalkylierung von Cyclen, die entsprechenden N-β-Hydroxyalkyl-1,4,7,10-tetraazacyclododecan- Lithium-Salz-Komplexe als Zwischenprodukte im Verfahren und die Verwendung der Komplexe zur Herstellung von Gadobutrol [Gd-Kompfexes von N-(1-Hydroxy-methyl-2,3-dihydroxypropyl)-1,4,7-triscarboxymethyl-1,4,7,10-tetraazacyclododecan] und Analoga.

Mono- und disubstituierte N-β-Hydroxyalkyl-1,4,7,10-tetraazacyclododecane sind wertvolle Zwischenstufen bei der Herstellung von NMR-Diagnostika (siehe DE A1 36 25 417, EP 0596586, EP 545 511 A2 und EP 0 448 191 A1). Die Synthese dieser Verbindungen erfolgt durch Umsetzung von 1,4,7,10-Tetraazacyclododecan (Cyclen) mit Epoxiden, wobei ein statistisches und schwer zu trennendes Gemisch von mono-, bis- und tris-alkylierten Produkten entsteht (WO93/24469). Durch Verwendung von großem Überschuß des teureren Polyazamakrocyclus kann zwar überwiegend das monoalkylierte Produkt erzeugt werden, die Trennung von dem überschüssigen Ausgangsmaterial ist aber weiterhin ein Problem, speziell bei der großtechnischen Herstellung (Mengen über 50 kg).

Die bekannten Methoden zur Herstellung von N-β-Hydroxyalkyl-1,4,7,10-tetraazacyclododecanen erfordert selektives Schützen der drei Stickstoffatome durch Umsetzung mit DMF-Dimethylacetal, mit Boranen (H. Bernard et al. Tetrahedron Lett. **1991,** 639) oder mit Cr- bzw. Mo-Carbonylkomplexen (J.-J. Yaouanc et al., Chem. Commun. **1991,** 206); daran schließt sich die Funktionalisierung des vierten Stickstoffatoms und anschließende Spaltung der Schutzgruppe an. Diese Methoden sind sehr aufwendig durchführbar und großtechnisch schwer anwendbar.
EP 0485045 und WO-A-94/27977 beschreiben die Epoxid-Alkylierung von tri-N-substituierten 1,4,7,10-Tetraazacyclododecanen.
Das in der US PS 5,064,633 beschriebene Verfahren zur Monoalkylierung von Polyazamakrocyclen ist auf die Verwendung von α-Halogencarbonsäurederivaten begrenzt und kann nicht für die Umsetzung mit Epoxiden zur Herstellung der gewünschten substituierten N-β-Hydroxyalkyl-1,4,7,10-tetraazacyclododecane angewendet werden.

Die regioselektive Formylierung von monoalkylierten Cyclenderivaten wurde von P. Anelli et al (J.Chem. Soc., Chem. Commun. **1991**, 1317) und die Herstellung von 1,7-disubstituierten Cyclenderivate durch Umsetzung mit Chlormeisensäureestern in J.Chem. Soc., Chem. Commun.**1995,** 185, beschrieben.

Aufgabe der Erfindung ist es, ein Verfahren zur direkten Mono- und 1,7-Bis-N-β-Hydroxyalkylierung von Cyclen bereitzustellen, das einen aufwendigen und mehrstufigen Schutz des Cyclens vermeidet.

Gelöst wird diese Aufgabe durch das erfindungsgemäße Verfahren zur Herstellung der Verbindungen der allgemeinen Formel I 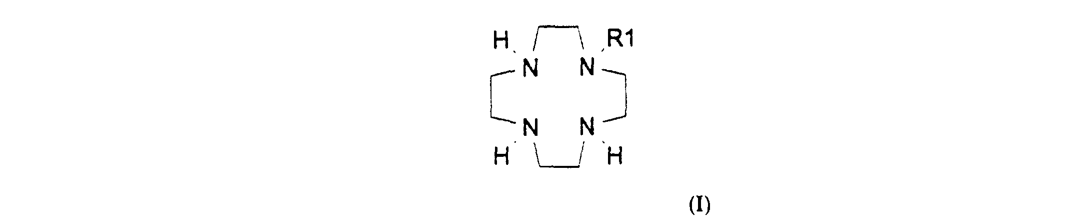 worin
R1 die Gruppe 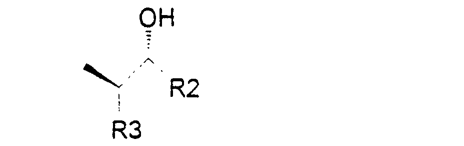 bedeutet und worin innerhalb von R¹ die Reste R² und R³ unabhängig voneinander jeweils für ein Wasserstoffatom stehen oder einen 4-, 5-, 6- oder 7-gliedrigen Cycfoalkyfring bilden, der gegebenenfalls durch 1 bis 3 Sauerstoffatom(e) unterbrochen sein kann, oder einen C₁-C₁₂ Alkylrest, der gegebenenfalls mit 1 bis 3 C₁-C₆-Alkyl- oder 1 bis 3 Hydroxygruppen substituiert ist, beispielsweise Hydroxymethyl, Hydroxyethyl, Methyl, Ethyl, Propyl, Butyl, wobei die vorhandene Hydroxyreste gegebenenfalls in geschützter Form vorliegen, durch Umsetzung von 1,4,7,10-Tetraazacyclododecan,
gegebenenfalls in Form eines Salzes,
mit einem Epoxid der Formel II, 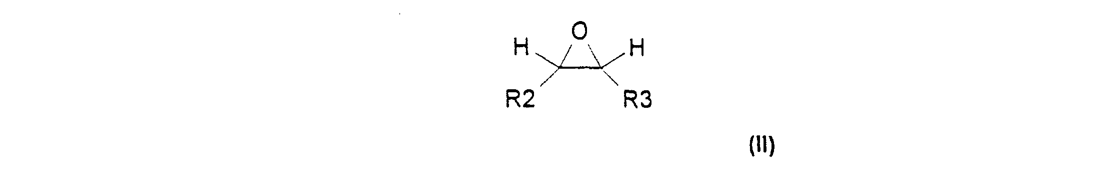 worin R² und R³ die oben angegebenen Bedeutungen haben, mit 0,8 - 1,1 mol, vorzugsweise mit 0,9 - 1,0 mol Lithiumsalz, wie beispielsweise Lithiumchlorid bei Temperaturen zwischen 40 - 150° C umsetzt und die erhaltene Reaktionsmischung gegebenenfalls wäßrig aufarbeitet.

Gelöst wird die Aufgabe der Erfindung weiter durch ein Verfahren zur Herstellung der Verbindungen der allgemeinen Formel lll

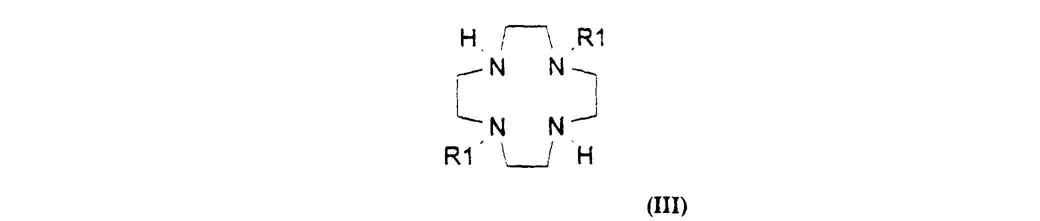

Die Verbindungen der allgemeinen Formel (III) lassen sich in ähnlicher Weise herstellen, wie die Verbindungen der allgemeinen Formel (I), mit dem Unterschied, daß mehr als 1,11 mol, vorzugsweise 1,5 - 3,0 mol Lithiumsalz (Lithiumchlorid) zugesetzt wird. Die Reste R¹ haben dieselbe Bedeutung, wie diese innerhalb der Verbindungen der allgemeinen Formel (I).

Durch die Zugabe von unterschiedlichen Mengen an Lithiumsalz (beispielsweise Lithiumchlorid) läßt sich die Selektivität der Alkylierung steuern. Beim Umsatz von 1 mol Cyclen mit bis zu 1,1 mol Lithiumsalz erhält man bis in zu 80 % das monoalkylierte Produkt, mit sehr geringem Anteil des 1,4-disubstituiertes Produktes. Setzt man mehr als 1,11 mol, vorzugsweise 2 und mehr Lithiumsalz pro Mol Cyclen in die Reaktion ein, so erhält man zu über 80 % das 1,7-disubstituierte Produkt, wenn mehr als 2 Mol Epoxid der allgemeinen Formel (III) verwendet werden. Steigert man die Menge des Epoxids, beispielsweise über 5 auf 10 Mol pro Mol Cyclen, so bleibt die Selektivität der Reaktion zu dem 1,7-disubstituierten Produkt erhalten.

Die folgenden Epoxide der allgemeinen Formel II eignen sich mit Vorteil für die Umsetzung im erfindungsgemäßen Verfahren: 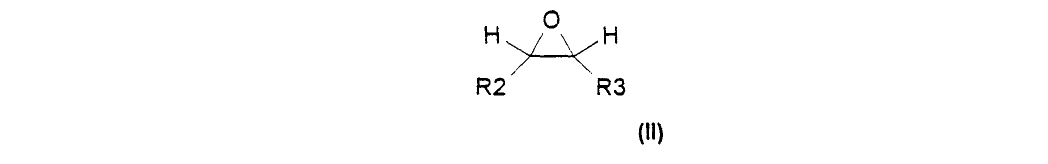
R² und R³ in der Bedeutung von Wasserstoff, Methyl, Propyl, Butyl oder höheres Alkyl, das gegebenenfalls durch eine oder mehrere Hydroxylgruppen substituiert sein kann, R² und
R³ zusammen einen 4-, 5-, 6- oder 7-gliedrigen Ring bilden, der durch 1-3 Sauerstoffatome unterbrochen sein kann, mit besonderem Vorteil eignen sich Cyclopenten-, Cyclohexen-, Cyclohepten-Epoxid, Ethylenoxid, Propylenoxid,1,2-Butenoxid oder 4,4-Dimethyl-3,5,8-trioxabicyclo[5,1,0]octan.

Die so erhaltene Rohprodukte, die zu über 80% aus dem gewünschten Mono-N-β- und 1,7-Bis N-β-Hydroxyalkyl-1,4,7,10-tetraazacyclododecan im Form von Lithium-Komplexen bestehen, können direkt zur Herstellung von NMR-Kontrastmitteln verwendet werden.

Als Lithiumsalze können die Chloride, Bromide, lodide, Perchlorate oder Trifluoracetate eingesetzt werden. Als Lösungsmittel können Dimethylforamid, Dimethoxyethan, Diethylenglykol-dimethylether, Toluol oder Alkohole, wie z.B. Isopropanol oder Gemische dieser Lösungsmittel verwendet werden. Das erfindungsgemäße Verfahren wird bei Temperaturen zwischen 40 und 150 °C, bevorzugt im Temperaturbereich 80 - 140° C, durchgeführt, die Reaktionszeit beträgt 20-50 Stunden.

Die Erfindung beinhaltet auch die Lithiumkomplexe der Verbindungen der allgemeinen Formeln (I) und (III); diese werden durch direkte Kristallisation des Reaktionsproduktes erhalten. Arbeitet man unter Zugabe von Wasser das Reaktionsgemisch auf, so erhält man die freien Verbindungen der allgemeinen Formeln (I) und (III).

Die Erfindung wird durch die nachfolgenden Beispiele erläutert:

### Beispiel 1:

### N-(6-Hydroxy-2,2-dimethyl-1,3-dioxepan-5-yl)-1,4,7,10-tetraazacyclododecan LiCI-Komplex

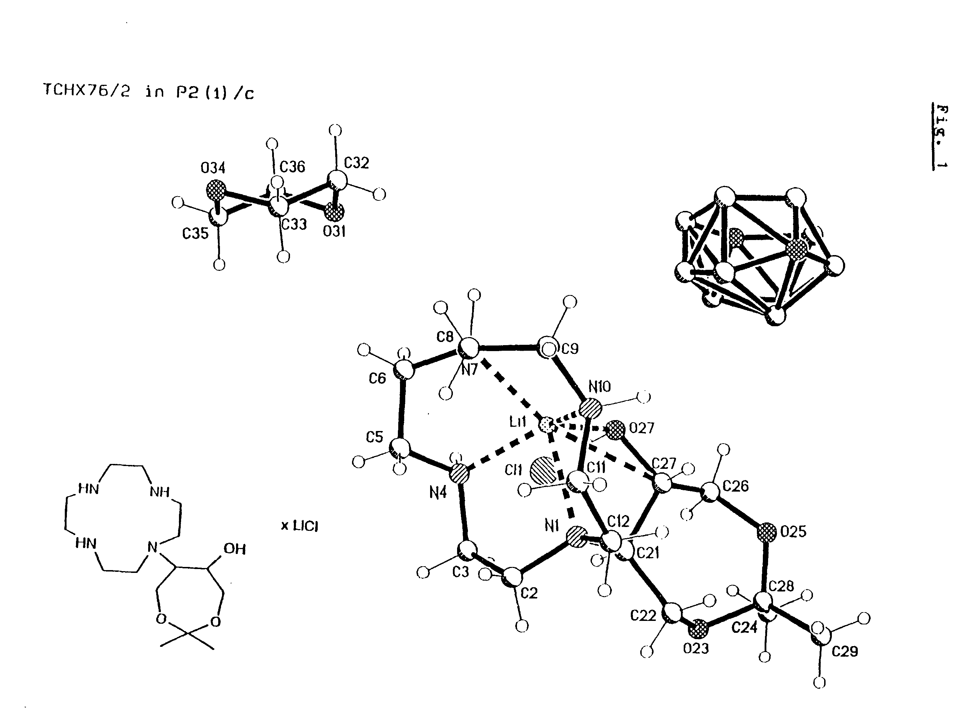

20g (116,16 mmol) 1,4,7,10-Tetraazacyclododecan, 4,85 g (116,16 mmol) LiCl und 19,17 g (133,58 mmol) 4,4-Dimethyl-3,5,8-trioxabicyclo[5,1,0]octan werden in 30 ml Isopropanol gelöst und 22 Stunden am Rückflüß gekocht. Anschließend wird das Reaktionsgemisch im Vakuum eingeengt und das erhaltene Rohprodukt (45,26 g) aus 150 ml MTB-Ether kristallisiert. Es werden 33,9 g (82% d.Th.) N-(6-Hydroxy-2,2-dimethyl-1,3-dioxepan-5-yl)-1,4,7,10-tetraazacyclododecan, LiCl-Komplex als weiße Kristalle erhalten. Schm. P. 197-199 °C. ¹H-NMR (DMSO-d₆): 3.65-3.40 m (5 H, CH₂-O, CH-O); 2,80-2.35 m (17 H, CH₂-N, CH-N), 1.23 s, (6H, CH₃). (**X-Ray: Fig. 1**)

### Beispiel 2.:

### 1,7-Bis((6-Hydroxy-2,2-dimethyl-1,3-dioxepan-5-yl)-1,4,7,10-tetraazacyclododecan LiCI-Komplex

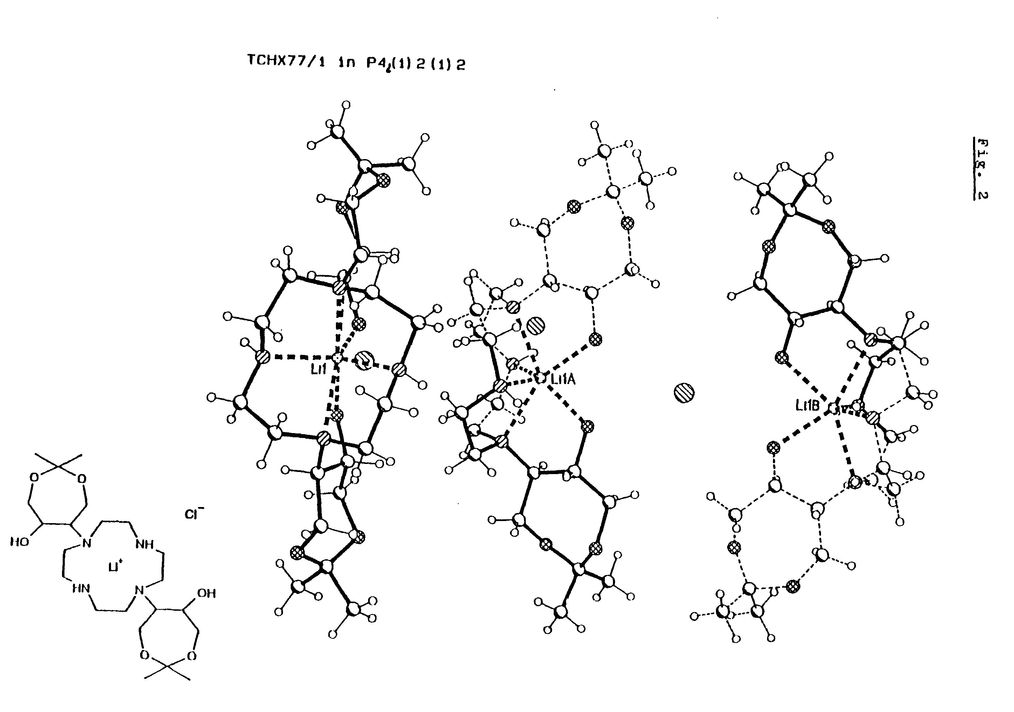

10g (58,08 mmol) 1,4,7,10-Tetraazacyclododecan, 4,85 g (116,16 mmol) LiCI und 40,0 g (278,4 mmol) 4,4-Dimethyl-3,5,8-trioxabicyclo[5,1,0]octan werden in 40 ml Isopropanol suspendiert und 60 Stunden am Rückfluß gekocht. Anschließend wird das Reaktionsgemisch mit 30 ml isopropanol versetzt und langsam auf 0 °C abgekühlt. Die erhaltenen Kristalle werden abgesaugt, mit 30 ml Methyl-tert.-butyl-ether gewaschen und im Vakuum getrocknet. Es werden 20,7 g (71% d.Th.) 1,7-Bis(6-hydroxy-2,2-dimethyl-1,3-dioxepan-5-yl)-1,4,7,10-tetraazacyclododecan LiCl-Komplex als weiße Kristalle erhalten. Fp.262-265 °C. ¹H-NMR (CD₃OD): 3.75-3.45 m (10 H, CH₂-O, CH-O); 2,97-2.30 m (18 H, CH₂-N, CH-N), 1.29 s, (12H, CH₃). **X-Ray: Fig 2.**

### Beispiel 3:

### N-(6-Hydroxycyclohehyl)-1,4,7,10-tetraazacyclododecan LiCl-Komplex:

1g (5,8 mmol) 1,4,7,10-Tetraazacyclododecan, 0,24 g (5,8 mmol) LiCI und 0,65 g (6,7 mmol) Cyclohexenoxid (7-Oxabicyclo[4.1.0]heptan) werden in 2,5 ml isopropanol gelöst und 18 Stunden am Rückfluß gekocht. Anschließend wird das Reaktionsgemisch im Vakuum eingeengt. Das Rohprodukt wird aus Methyl-tert.-butyl-ether kristallisiert. Es werden 0,95 g (52 % d. Th.) N-(6-Hydroxycyclohehyl)-1,4,7,10-tetraazacyclododecan LiCI-Komplex als farblose Kristalle erhalten. Schm. P. 130-135 °C. ¹H-NMR (DMSO-d₆): 3.50-3.45 m (1 H, CH-O); 2,75-2.30 m (17 H, CH₂-N, CH-N), 2,00-1,57m (4H, CH₂-CH₂); 1,30-1,08m (4H, CH₂-CH₂).

### Beispiel 4:

### 1,7-Bis(6-Hydroxycyclohexyl)-1,4,7,10-tetraazacyclododecan LiCl-Komplex:

10g (58,08 mmol) 1,4,7,10-Tetraazacyclododecan, 4,85 g (116,16 mmol) LiCI und 56,5 g (580,0 mmol) Cyclohexenoxid (7-Oxabicyclo[4.1.0]heptan) werden in 20 ml Isopropanol suspendiert und 20 Stunden am Rückflüß gekocht. Anschließend wird das Reaktionsgemisch mit 30 ml Isopropanol versetzt und langsam auf 0 °C abgekühlt. Die erhaltenen Kristalle werden abgesaugt, mit 30 ml Methyl-tert.-butyl-ether gewaschen und und im Vakuum getrocknet. Es werden 19,5 g (82% d.Th.) 1,7-Bis(6-hydroxycyclohexyl)-1,4,7,10-tetraazacyclododecan LiCI-Komplex als weiße Kristalle erhalten. Schmp.170 - 178°C.
¹H-NMR (DMSO-d₆ 80 °C): 3.48-3.25 m (2 H, CH-O); 2,85-2.20 m (18 H, CH₂-N, CH-N), 2,03-1,50 m (8H, CH₂-CH₂); 1,30-1,00 m (8H, CH₂-CH₂). MS (FAB): 369 [M+H]⁺.

### Beispiel 5:

### Gd-Komplex von N-(1-Hydroxymethyl-2,3-dihydroxypropyl)-1,4,7-triscarboxymethyl-1,4,7,10-tetraazacyclododecan

Das aus Beispiel 1 erhaltene Rohprodukt wird in 50 ml Wasser gelöst und bei 70 °C mit einer Lösung von 38,41 g (406,56 mmol) Chloressigsäure und 16,26 g (406,56 mmol) NaOH in 45 ml Wasser versetzt. Das Reaktionsgemisch wird 6 Stunden bei 65 °C gerührt, während dessen der pH-Wert mit NaOH bei 11,0 gehalten wird. Anschließend wird mit konz. HCl auf pH 1,5 angesäuert und das Reaktionsgemisch im Vakuum eingeengt. Der Rückstand wird mit 500 ml Methanol aufgenommem und die ungelösten Salze abfiltriert. Das Filtrat wird eingeengt, in 500 ml Wasser gelöst, mit 18,91 g (52,27 mmol) Gadoliniumoxid versetzt und 2 Studen bei 100 °C gerührt. Anschließend wird mit LiOH neutral gestellt und das Wasser im Vakuum eingeengt. Der Rest wird aus Ethanol/Wasser kristallisiert. Es werden 60,92 g kristallines Rohprodukt erhalten. Das Produkt wird in 2 l Wasser gelöst und durch Behandlung mit 500 ml Amberlite IRA 67 und 500 ml IRC 50 von ionischen Verunreinigungen befreit. Anschließend wird die Lösung eingeengt und der Rückstand aus EtOH/Wasser umkristallisiert.
Es werden 40,74 g (58% d.Th., bezogen auf das Einsatzmaterial) des Gd-Komplexes von N-(1-Hydroxy-methyl-2,3-dihydroxypropyl)-1,4,7-triscarboxymethyl-1,4,7,10-tetraazacyclododecan erhalten. Das so erhaltene Rohprodukt läßt sich so ohne weitere Reinigungsschritte analog der in DE 42 18 744 A1 enthaltenen Vorschrift in das Gadobutrol umsetzen.

### Beispiel 6:

### N-(6-Hydroxycyclohehyl)-1,4,7,10-tetraazacyclododecan:

1g (5,8 mmol) 1,4,7,10-Tetraazacyclododecan, 0,24 g (5,8 mmol) LiCl und 0,65 g (6,7 mmol) Cyclohexenoxid (7-Oxabicyclo[4.1.0]heptan) werden in 2,5 ml Isopropanol gelöst und 18 Stunden am Rückfluß gekocht. Anschließend wird das Reaktionsgemisch im Vakuum eingeengt. Das Rohprodukt wird in 10 ml Chloroform aufgenommen und mit 5 ml Wasser extrahiert. Die organische Phase wird eingeengt. Es werden 1,25 g (79 5 d. Th.) N-(6-Hydroxycyclohehyl)-1,4,7,10-tetraazacyclododecan als farbloses Öl erhalten.

## Patentansprüche

1. Verfahren zur Herstellung der Verbindungen der allgemeinen Formel l 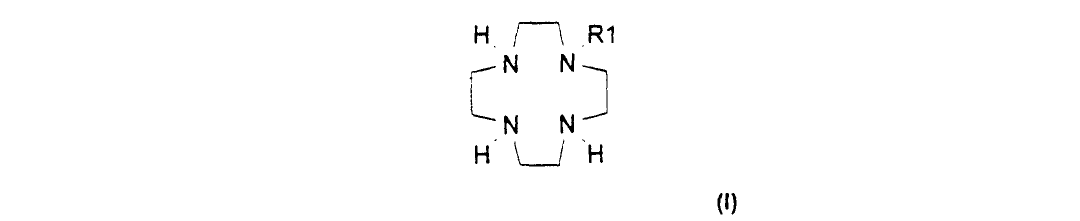 und R1 die Gruppe 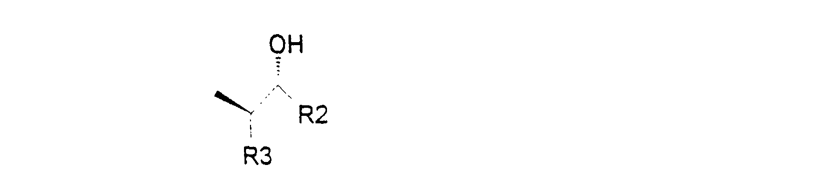 bedeutet und
worin innerhalb von R¹ die Reste
R² und R³ unabhängig voneinander jeweils für ein Wasserstoffatom stehen oder einen 4-, 5-, 6- oder 7-gliedrigen Cycloalkylring bilden, der gegebenenfalls durch 1 bis 3 Sauerstoffatom(e) unterbrochen sein kann, oder einen C₁-C₁₂ Alkylrest, der gegebenenfalls mit 1 bis 3 C₁-C₆-Alkyloder 1 bis 3 Hydroxygruppen substituiert ist,
wobei die gegebenenfalls vorhandenen Hydroxylreste gegebenenfalls in geschützter Form vorliegen, durch Umsetzung von 1,4,7,10-Tetraazacyclodo-decan , gegebenenfalls in Form eines Salzes, mit einem Epoxid der Formel II, 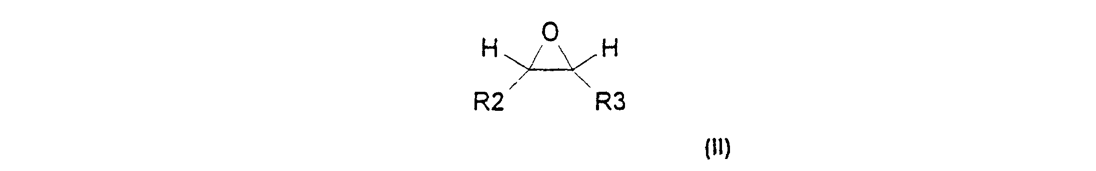 worin
R² und R³ die oben angegebenen Bedeutungen haben, wobei vorhandene Hydroxygruppen gegebenenfalls geschützt sind,
mit 0,8 - 1,1 mol, vorzugsweise 0,9 - 1,0 mol Lithiumsalz bezogen auf ein Mol Cyclen bei Temperaturen zwischen 40 - 150° C umsetzt und die Reaktion wäßrig aufarbeitet.

2. Verfahren zur Herstellung der Verbindungen der allgemeinen Formel lll 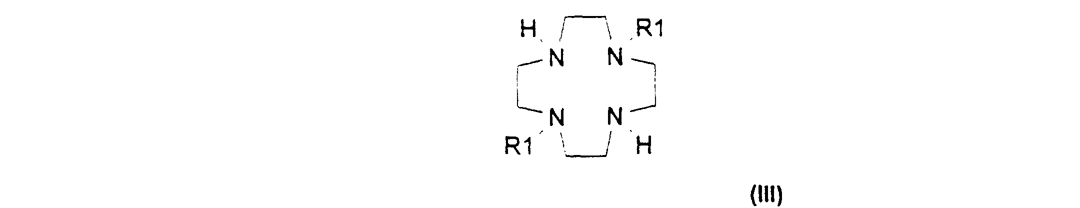 worin
R1 die Gruppe 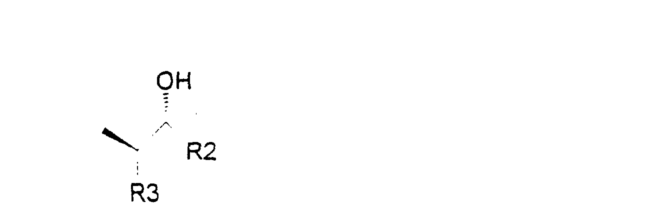 bedeutet und
worin innerhalb von R¹ die Reste
R² und R³ unabhängig voneinander jeweils für ein Wasserstoffatom stehen , oder einen 4-, 5-, 6- oder 7-gliedrigen Cycloalkylring bilden, der gegebenenfalls durch 1 bis 3 Sauerstoffatom(e) unterbrochen sein kann, oder einen C₁-C₁₂ Alkylrest, der gegebenenfalls mit 1 bis 3 C₁-C₆-Alkyloder 1 bis 3 Hydroxygruppen substituiert ist,
wobei die gegebenenfalls vorhandenen Hydroxyreste gegebenenfalls in geschützter Form vorliegen, durch Umsetzung von 1,4,7,10-Tetraazacyclodo-decan, gegebenenfalls in Form eines Salzes, mit einem Epoxid der Formel II, 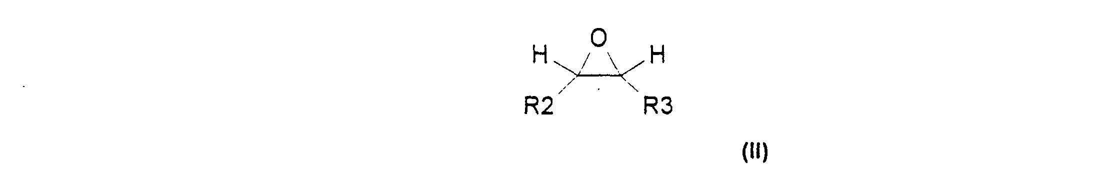 worin
R² und R³ die oben angegebenen Bedeutungen haben, wobei gegebenenfalls vorhandene Hydroxygruppen gegebenenfalls geschützt sind, mit mehr als 1,11 mol Lithiumsalz, bezogen auf 1 Mol Cyclen, vorzugsweise 2,0 - 3,0 mol Lithiumsalz bei Temperaturen zwischen 40 - 150° C umsetzt und die Reaktion wäßrig aufarbeitet.

3. Lithiumkomplexe der Verbindungen der allgemeinen Formel l gemäß Anspruch 1 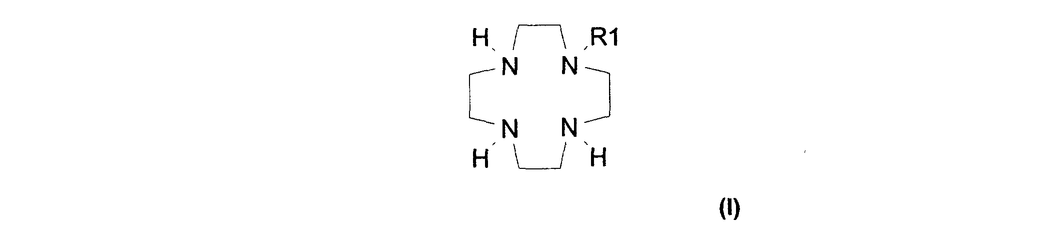 worin R1 die Gruppe 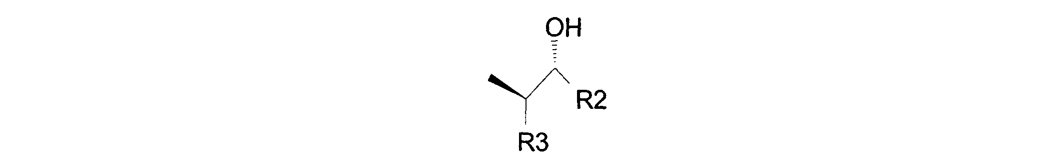 bedeutet und
worin innerhalb von R¹ die Reste
R² und R³ unabhängig voneinander jeweils für ein Wasserstoffatom stehen , oder einen 4-, 5-, 6- oder 7-gliedrigen Cycloalkylring bilden, der gegebenenfalls durch 1 bis 3 Sauerstoffatom(e) unterbrochen sein kann, oder einen C₁-C₁₂ Alkylrest, der gegebenenfalls mit 1 bis 3 C₁-C₆-Alkyloder 1 bis 3 Hydroxygruppen substituiert ist .

4. Lithiumkomplexe der Verbindungen der allgemeinen Formel III gemäß Anspruch 2 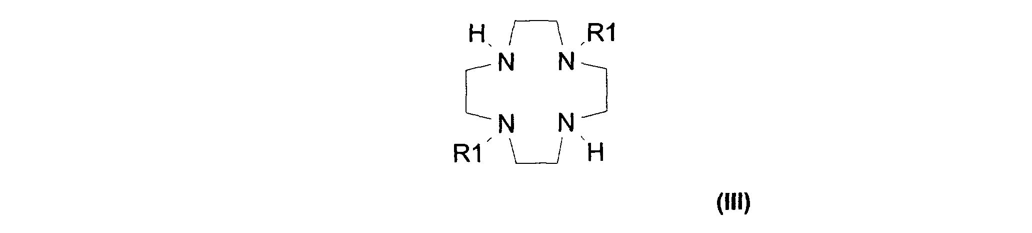 worin R1 die Gruppe 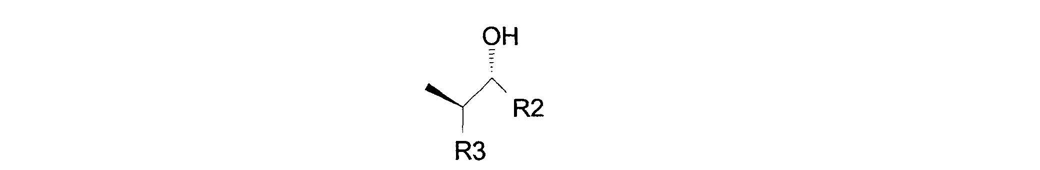 bedeutet und
worin innerhalb von R¹ die Reste
R² und R³ unabhängig voneinander jeweils für ein Wasserstoffatom stehen oder einen 4-, 5-, 6- oder 7-gliedrigen Cycloalkylring bilden, der gegebenenfalls durch 1 bis 3 Sauerstoffatom(e) unterbrochen sein kann, oder einen C₁-C₁₂ Alkylrest, der gegebenenfalls mit 1 bis 3 C₁-C₆-Alkyloder 1 bis 3 Hydroxygruppen substituiert ist .

5. Lithium-Komplexe gemäß Ansprüche 1 und 2:
1,7-Bis(6-Hydroxycyclohexyl)-1,4,7,10-tetraazacyclododecan LiCl-Komplex,
N-(6-Hydroxycyclohehyl)-1,4,7,10-tetraazacyclododecan LiCl-Komplex,
1,7-Bis((6-Hydroxy-2,2-dimethyl-1,3-dioxepan-5-yl)-1,4,7,10-tetraazacyclododecan LiCl-Komplex,
N-(6-Hydroxy-2,2-dimethyl-1,3-dioxepan-5-yl)-1,4,7,10-tetraazacyclododecan-LiCI-Komplex.

6. Verwendung der Lithiumkomplexe gemäß Ansprüche 3 und 4 zur Herstellung von Gadubutrol-Analoga.

7. Verwendung von N-(6-Hydroxy-2,2-dimethyl-1,3-dioxepan-5-yl)-1,4,7,10-tetraazacyclododecan-LiCI-Komplex gemäß Anspruch 1 zur Herstellung von Gadobutrol [Gd-Komplexes von N-(1-Hydroxy-methyl-2,3-dihydroxypropyl)-1,4,7-triscarboxymethyl-1,4,7,10-tetraazacyclododecan.

## Claims

1. Process for the production of compounds of general formula I 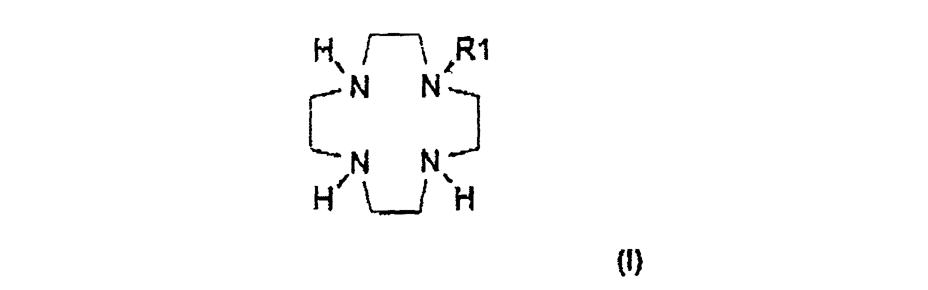 in which R1 denotes the group 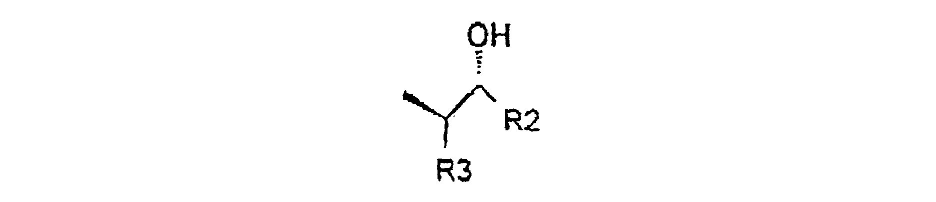 and
in which within R¹, radicals
R² and R³, independently of one another, in each case stand for a hydrogen atom or form a 4-, 5-, 6- or 7-membered cycloalkyl ring, which optionally can be interrupted by 1 to 3 oxygen atom(s), or a C₁-C₁₂ alkyl radical, which optionally is substituted with 1 to 3 C₁-C₆ alkyl groups or 1 to 3 hydroxy groups,
where the optionally present hydroxyl radicals are optionally present in protected form, by reaction of 1,4,7,10-tetraazacyclododecane, optionally in the form of a salt, with an epoxide of formula II, 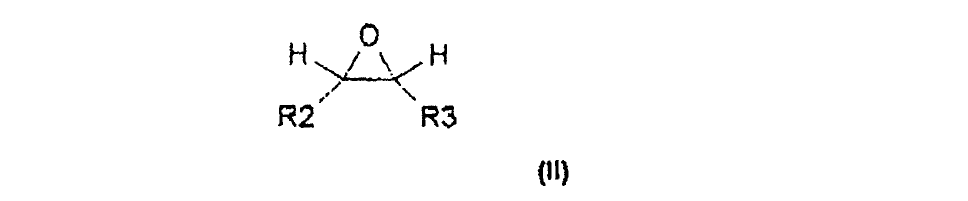 in which
R² and R³ have the above-indicated meanings, where hydroxy groups that are present are optionally protected
in the presence of 0.8-1.1 mol, preferably 0.9-1.0 mol, of lithium salt relative to one mol of cyclene at temperatures between 40-150°C, and the reaction is worked up in aqueous form.

2. Process for the production of compounds of general formula III 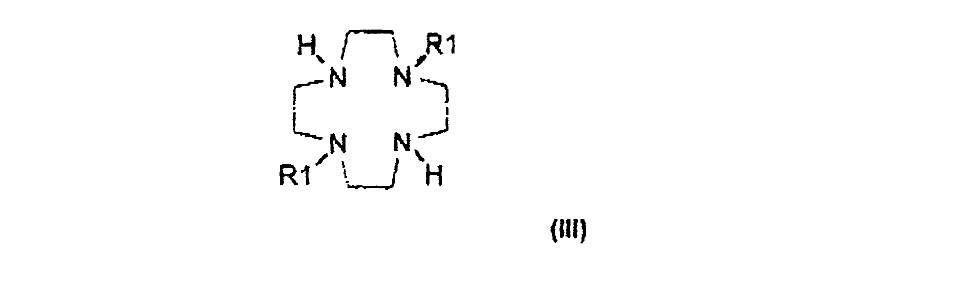 in which R1 denotes the group 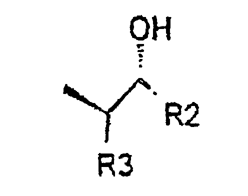 and
in which within R¹, radicals
R² and R³, independently of one another, in each case stand for a hydrogen atom or form a 4-, 5-, 6- or 7-membered cycloalkyl ring, which optionally can be interrupted by 1 to 3 oxygen atom(s), or a C₁-C₁₂ alkyl radical, which optionally is substituted with 1 to 3 C₁-C₆ alkyl groups or 1 to 3 hydroxy groups,
where the optionally present hydroxyl radicals are optionally present in protected form, by reaction of 1,4,7,10-tetraazacyclododecane, optionally in the form of a salt, with an epoxide of formula II, 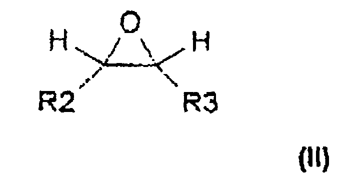 in which
R² and R³ have the above-indicated meanings, where optionally present hydroxy groups are optionally protected
in the presence of more than 1.11 mol of lithium salt relative to 1 mol of cyclene, preferably 2.0-3.0 mol of lithium salt, at temperatures of from 40-150°C, and the reaction is worked up in aqueous form.

3. Lithium complexes of compounds of general formula I according to Claim 1 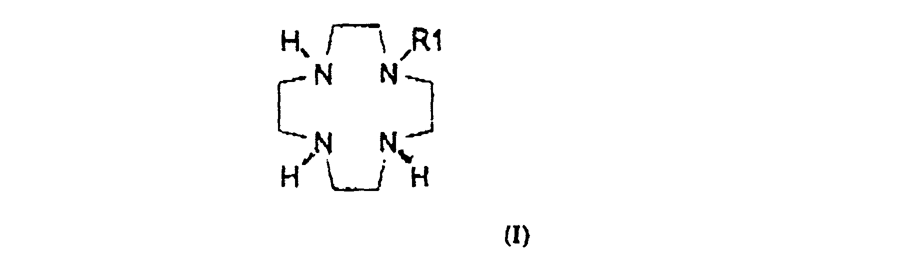 in which R1 denotes the group 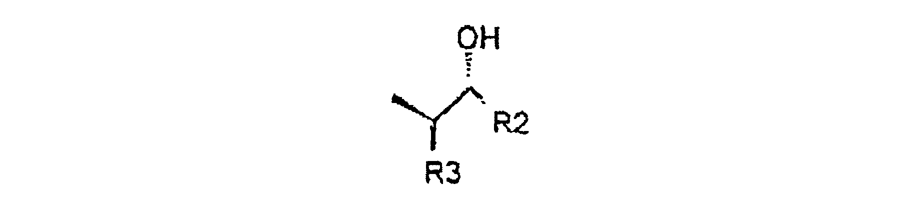 and
in which within R¹, radicals
R² and R³, independently of one another, in each case stand for a hydrogen atom or form a 4-, 5-, 6- or 7-membered cycloalkyl ring, which optionally can be interrupted by 1 to 3 oxygen atom(s), or a C₁-C₁₂ alkyl radical, which optionally is substituted with 1 to 3 C₁-C₆ alkyl groups or 1 to 3 hydroxy groups.

4. Lithium complexes of compounds of general formula III according to Claim 2 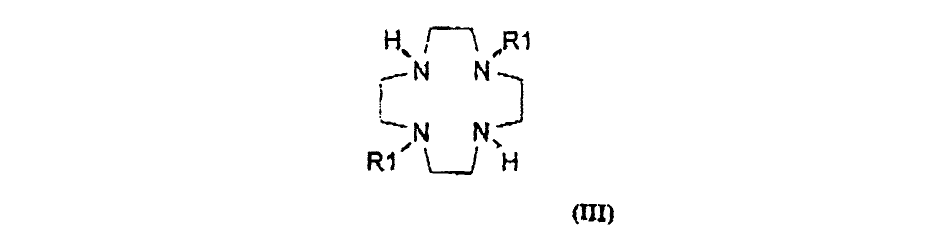 in which R1 denotes the group 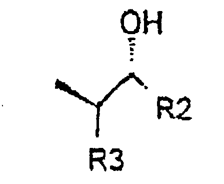 and
in which within R¹, radicals
R² and R³, independently of one another, in each case stand for a hydrogen atom or form a 4-, 5-, 6- or 7-membered cycloalkyl ring, which optionally can be interrupted by 1 to 3 oxygen atom(s), or a C₁-C₁₂ alkyl radical, which optionally is substituted with 1 to 3 C₁-C₆ alkyl groups or 1 to 3 hydroxy groups.

5. Lithium complexes according to Claims 1 and 2:
1,7-bis(6-hydroxycyclohexyl)-1,4,7,10-tetraazacyclododecane LiCl complex,
N-(6-hydroxycyclohexyl)-1,4,7,10-tetraazacyclododecane LiCl complex,
1,7-bis((6-hydroxy-2,2-dimethyl-1,3-dioxepan-5-yl)-1,4,7,10-tetraazacyclododecane LiCl complex,
N-(6-hydroxy-2,2-dimethyl-1,3-dioxepan-5-yl)-1,4,7,10-tetraazacyclododecane LiCl complex.

6. Use of the lithium complexes according to Claims 3 and 4 for the production of gadobutrol analogues.

7. Use of N-(6-hydroxy-2,2-dimethyl-1,3-dioxepan-5-yl)-1,4,7,10-tetraazacyclododecane LiCl complex according to Claim 1 for the production of gadobutrol (Gd complex of N-(1-hydroxymethyl-2,3-dihydroxypropyl)-1,4,7-triscarboxymethyl-1,4,7,10-tetraazacyclododecane.

## Revendications

1. Procédé de préparation des composés de formule générale I 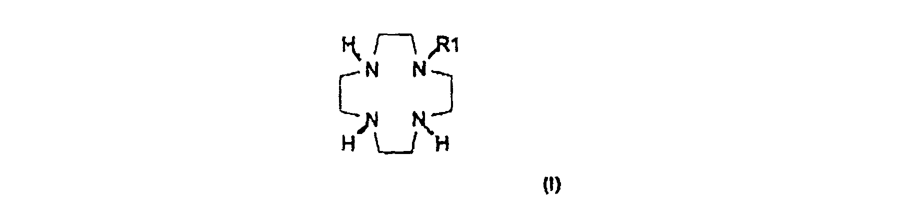 dans laquelle R¹ représente le groupe 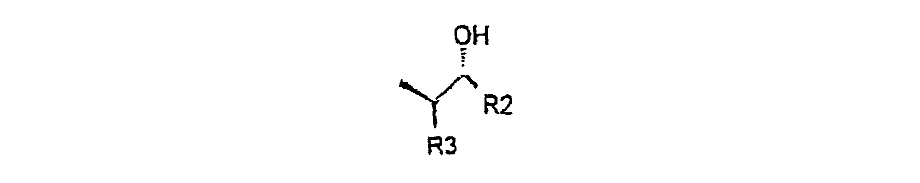 et, à l'intérieur de R¹, les radicaux
R² et R³ représentent chacun, indépendamment l'un de l'autre, un atome d'hydrogène ou forment un noyau cycloalkyle à 4, 5, 6 ou 7 chaînons, lequel peut éventuellement être interrompu par de 1 à 3 atome(s) d'oxygène, ou un radical alkyle en C₁-C₁₂, qui est éventuellement substitué par de 1 à 3 groupes alkyle en C₁-C₆ ou de 1 à 3 groupes hydroxy,
les radicaux hydroxy éventuellement présents se trouvant éventuellement sous forme protégée, par réaction du 1,4,7,10-tétraazacyclododécane, éventuellement sous la forme d'un sel, avec un époxyde de formule II, 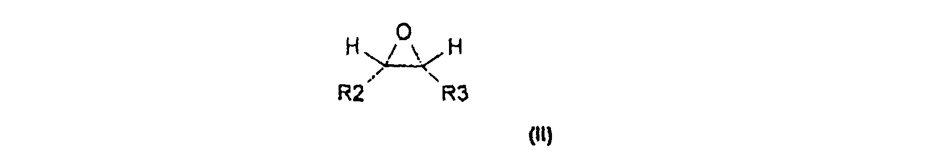 dans laquelle
R² et R³ présentent les significations indiquées ci-dessus, les groupes hydroxy présents étant éventuellement protégés,
avec de 0,8 à 1,1 mol, de préférence de 0,9 à 1,0 mol d'un sel de lithium, par rapport à une mole de cyclène, à des températures comprises entre 40 et 150°C, et la réaction est traitée en phase aqueuse.

2. Procédé de préparation des composés de formule générale III 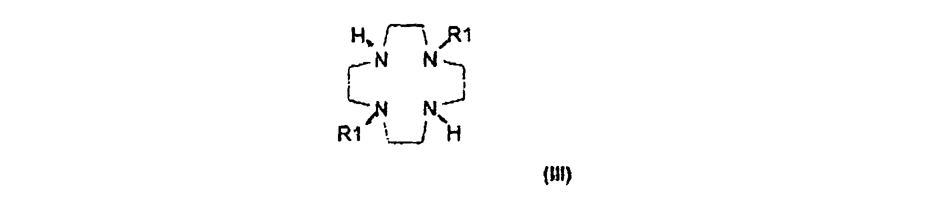 dans laquelle
R¹ représente le groupe 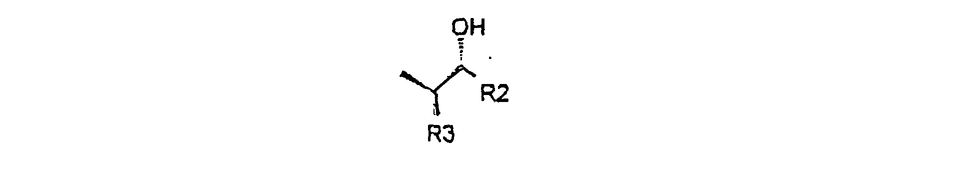 et, à l'intérieur de R¹, les radicaux
R² et R³ représentent chacun, indépendamment l'un de l'autre, un atome d'hydrogène ou forment un noyau cycloalkyle à 4, 5, 6 ou 7 chaînons, lequel peut éventuellement être interrompu par de 1 à 3 atome(s) d'oxygène, ou un radical alkyle en C₁-C₁₂, qui est éventuellement substitué par de 1 à 3 groupes alkyle en C₁-C₆ ou de 1 à 3 groupes hydroxy,
les radicaux hydroxy éventuellement présents se trouvant éventuellement sous forme protégée, par réaction du 1,4,7,10-tétraazacyclododécane, éventuellement sous la forme d'un sel, avec un époxyde de formule II, 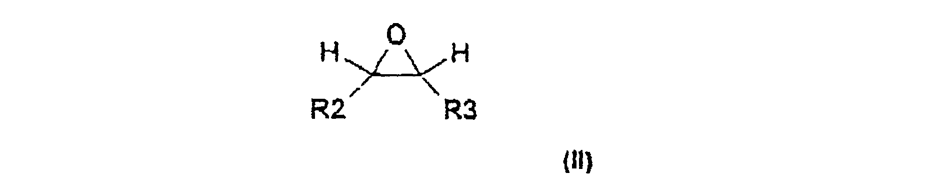 dans laquelle
R² et R³ présentent les significations indiquées ci-dessus, les groupes hydroxy éventuellement présents étant éventuellement protégés,
avec plus de 1,11 mol d'un sel de lithium, par rapport à un 1 mol de cyclène, de préférence de 2,0 à 3,0 mol d'un sel de lithium, à des températures comprises entre 40 et 150°C, et la réaction est traitée en phase aqueuse.

3. Complexes de lithium des composés de formule générale I selon la revendication 1 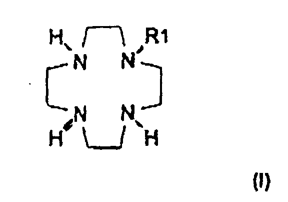 dans laquelle R¹ représente le groupe 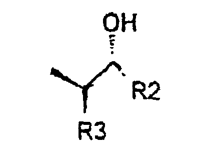 et, à l'intérieur de R¹, les radicaux
R² et R³ représentent chacun, indépendamment l'un de l'autre, un atome d'hydrogène ou forment un noyau cycloalkyle à 4, 5, 6 ou 7 chaînons, lequel peut éventuellement être interrompu par de 1 à 3 atome(s) d'oxygène, ou un radical alkyle en C₁-C₁₂, qui est éventuellement substitué par de 1 à 3 groupes alkyle en C₁-C₆ ou de 1 à 3 groupes hydroxy.

4. Complexes de lithium des composés de formule générale III selon la revendication 2 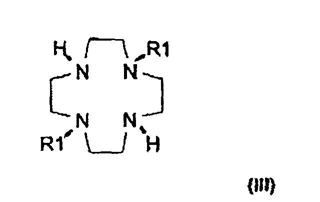 dans laquelle R¹ représente le groupe 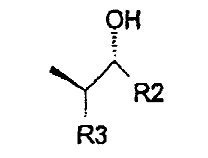 et, à l'intérieur de R¹, les radicaux
R² et R³ représentent chacun, indépendamment l'un de l'autre, un atome d'hydrogène ou forment un noyau cycloalkyle à 4, 5, 6 ou 7 chaînons, lequel peut éventuellement être interrompu par de 1 à 3 atome (s) d'oxygène, ou un radical alkyle en C₁-C₁₂, qui est éventuellement substitué par de 1 à 3 groupes alkyle en C₁-C₆ ou de 1 à 3 groupes hydroxy.

5. Complexes de lithium selon les revendications 1 et 2, à savoir :
le complexe LiCl de 1,7-bis(6-hydroxycyclohexyl)-1,4,7,10-tétraazacyclododécane,
le complexe LiCl de N-(6-hydroxycyclohexyl)-1,4,7,10-tétraazacyclododécane,
le complexe LiCl de 1,7-bis(6-hydroxy-2,2-diméthyl-1,3-dioxépan-5-yl)-1,4,7,10-tétraazacyclo-dodécane
le complexe LiCl de N-(6-hydroxy-2,2-diméthyl-1,3-dioxépan-5-yl)-1,4,7,10-tétraazacyclododécane.

6. Utilisation des complexes de lithium selon les revendications 3 et 4 pour la préparation d'analogues du Gadubutrol.

7. Utilisation du complexe LiCl de N-(6-hydroxy-2,2-diméthyl-3-dioxépan-5-yl)-1,4,7,10-tétraazacyclodo-décane selon la revendication 1 pour la préparation du Gadobutrol (complexe Gd de N-(1-hydroxyméthyl-2,3-dihydroxypropyl)-1,4,7-triscarboxyméthyl-1,4,7,10-tétraazacyclododécane].
